(19) Europäisches Patentamt
European Patent Office
Office européen des brevets

(11) EP 3 272 783 A1

(12) # DEMANDE DE BREVET EUROPEEN

(43) Date de publication:
**24.01.2018 Bulletin 2018/04**

(51) Int Cl.:
***C08G 61/08*** *(2006.01)* ***C08G 61/12*** *(2006.01)*
***C09J 165/00*** *(2006.01)* ***C09J 177/00*** *(2006.01)*

(21) Numéro de dépôt: **17180763.9**

(22) Date de dépôt: **11.07.2017**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**MA MD**

(30) Priorité: **19.07.2016 FR 1656864**

(71) Demandeurs:
- **BOSTIK SA**
  **93210 La Plaine Saint Denis (FR)**
- **Université de Rennes 1**
  **35065 Rennes Cedex (FR)**
- **CENTRE NATIONAL DE LA RECHERCHE SCIENTIFIQUE**
  **75016 Paris (FR)**

(72) Inventeurs:
- **MICHAUD, Guillaume**
  **60200 COMPIEGNE (FR)**
- **SIMON, Frédéric**
  **60400 PONT L'EVEQUE (FR)**
- **FOUQUAY, Stéphane**
  **76130 MONT SAINT-AIGNAN (FR)**
- **VANBIERVLIET, Elise**
  **35000 RENNES (FR)**
- **GUILLAUME, Sophie**
  **35500 VITRE (FR)**
- **CARPENTIER, Jean-François**
  **35690 ACIGNE (FR)**

(74) Mandataire: **Granet, Pierre**
**ARKEMA France**
**Département Propriété Industrielle**
**420, rue d'Estienne d'Orves**
**92705 Colombes Cedex (FR)**

(54) **POLYMÈRES HYDROCARBONÉS À DEUX GROUPEMENTS TERMINAUX AZLACTONES**

(57) **1)** Polymère hydrocarboné comprenant deux groupements terminaux azlactones de formule (I) :

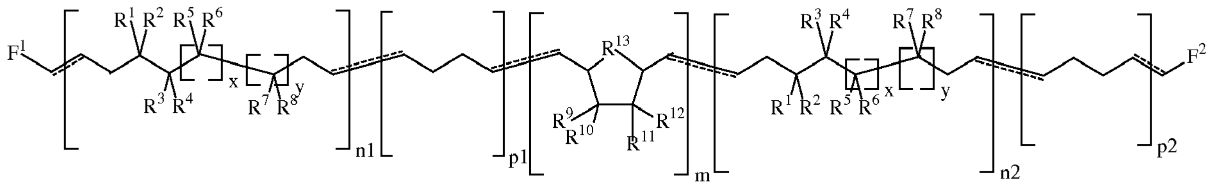

dans laquelle $F^1$ représente un radical de formule (IIa) et $F^2$ représente un radical de formule (IIb):

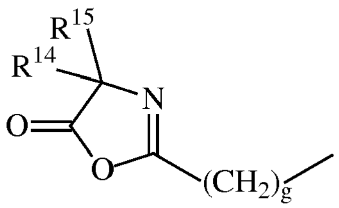

(IIa)

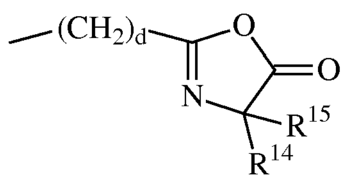

(IIb)

dans lesquelles :
- g et d sont un nombre entier égal à 0, 1, 2 ou 3 ;
- $R^{14}$ et $R^{15}$ représentent un radical $C_1$-$C_4$ alkyle ou bien forment avec l'atome de carbone auxquels ils sont reliés un radical cyclohexyle ;
- $R^1$ à $R^{12}$ représentent un atome d'hydrogène ou un radical alkyle comprenant de 1 à 22 atomes de carbone ;

EP 3 272 783 A1

- x et y sont des nombres entiers tels que la somme x + y est comprise dans une fourchette allant de 0 à 2 ;
- $R^{13}$ est un atome d'oxygène, de soufre, ou un radical divalent $-CH_2$ ;
- n1, n2, m, p1 et p2 sont un nombre entier ou égal à 0 et tels que la masse moléculaire Mn du polymère de formule (I) est comprise entre 400 à 100 000 g/mol.

**2)** Procédé de préparation dudit polymère par polymérisation par ouverture de cycle par métathèse
**3)** Utilisation comme adhésif en mélange avec un composé aminé comprenant au moins deux groupements amines.

## Description

La présente invention a pour objet des polymères hydrocarbonés comprenant deux groupements terminaux azlactones (également dénommés oxazolones), leur préparation et leur utilisation comme adhésif.

Les polyuréthanes sont largement utilisés dans le domaine des adhésifs, en raison de la polyvalence de leurs propriétés, rendue possible par leur nombre très élevé de formes structurales.

La synthèse des polyuréthanes se fait traditionnellement par réaction entre un diol et un diisocyanate. Les diisocyanates sont des composés toxiques en tant que tels, et sont généralement obtenus à partir de phosgène, lui-même très toxique par inhalation ou par contact. Le procédé de fabrication utilisé dans l'industrie met généralement en oeuvre la réaction d'une amine avec un excès de phosgène pour former un isocyanate.

La recherche d'alternatives à la synthèse de polyuréthanes sans utiliser d'isocyanate (ou NIPU pour « Non Isocyanate PolyUrethane » en anglais, c'est-à-dire « polyuréthane sans isocyanate »), représente donc un enjeu majeur pour l'industrie des adhésifs.

On peut citer comme exemple d'une telle approche la demande de brevet WO 2014/091173 au nom de Bostik et du CNRS, qui décrit des polymères hydrocarbonés comprenant deux groupements terminaux à terminaison (2-oxo-1,3-dioxolan-4-yl) susceptibles d'être obtenus par polymérisation par ouverture de cycle par métathèse, à partir d'au moins une cyclooléfine cyclique, au moins un agent de transfert de chaine insaturé non cyclique comprenant un groupement terminal (2-oxo-1,3-dioxolan-4-yl), et au moins un catalyseur de métathèse. Ces polymères peuvent ensuite réagir avec une (poly)amine pour former des polyuréthanes, sans mise en oeuvre d'isocyanate, qui peuvent être avantageusement utilisés pour formuler des compositions de revêtements, de mastics ou d'adhésifs. Toutefois, cette réaction est relativement longue et reste à améliorer.

C'est pourquoi, il peut être également envisagé comme alternative à cette approche d'envisager la recherche de nouvelles familles de composés à propriétés adhésives, qui ne nécessitent pas la mise en oeuvre d'isocyanates pour leur préparation et qui soient susceptibles de présenter la même polyvalence de propriétés que celle des polyuréthanes.

On connaît ainsi par la demande de brevet EP 0,105,665 des composés à groupements terminaux azlactones, qui sont susceptibles de réagir par ouverture du cycle azlactone, en présence de réactifs à groupe nucléophile comme les amines, pour former des polyamides thermoplastiques et thermodurs utiles notamment comme adhésifs.

Un but de la présente invention est de proposer de nouveaux composés à groupements terminaux azlactones, susceptibles d'offrir des propriétés avantageuses dans le domaine des adhésifs, par exemple en terme de propriétés mécaniques du joint adhésif et notamment de ses propriétés viscoélastiques.

Un autre but de la présente invention est de proposer des polymères à groupements terminaux azlactones et à chaîne principale hydrocarbonés, saturée ou insaturée, susceptibles d'être obtenus avec un rendement élevé.

Ainsi, la présente invention concerne un polymère hydrocarboné comprenant deux groupements terminaux azlactones (également dénommés oxazolones), ledit polymère hydrocarboné ayant la formule (1) :

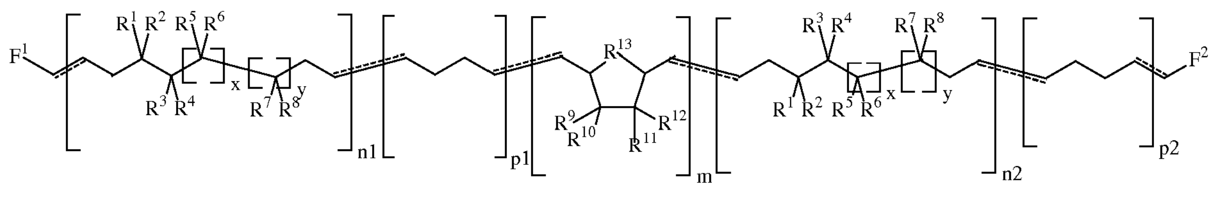

(I)

dans laquelle :

- $F^1$ représente un radical de formule (IIa) et $F^2$ représente un radical de formule (IIb):

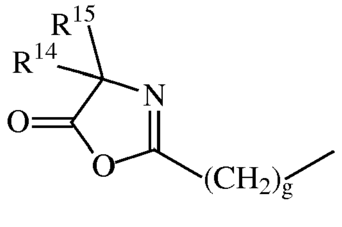

(IIa)

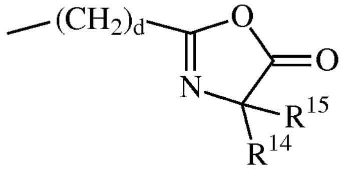

(IIb)

dans lesquelles :

- g et d, identiques ou différents, représentent un nombre entier égal à 0, 1, 2 ou 3 ;
- $R^{14}$ et $R^{15}$, identiques ou différents, représentent un atome d'hydrogène ou un groupement hydrocarboné aromatique ou aliphatique, linéaire ou ramifié, comprenant de 1 à 20 atomes de carbone pouvant être interrompu par un ou plusieurs atome d'oxygène ou de soufre ; en outre les groupes $R^{14}$ et $R^{15}$ peuvent former avec l'atome de carbone auxquels ils sont reliés un cycle hydrocarboné comprenant de 4 à 10 chaînons et éventuellement un ou plusieurs hétéroatomes choisis parmi l'oxygène et le soufre ;

- chaque liaison carbone - carbone de la chaîne principale du polymère, notée ------ représente une double liaison ou une simple liaison, conformément aux règles de valence de la chimie organique ;

- $R^1$, $R^2$, $R^3$, $R^4$, $R^5$, $R^6$, $R^7$ et $R^8$, identiques ou différents, représentent :

  - un atome d'hydrogène ou d'halogène ; ou
  - un radical comprenant de 1 à 22 atomes de carbone choisi parmi alkyle, alcényle, alcoxycarbonyle, alcényloxycarbonyle, alkylcarbonyloxy, alcénylcarbonyloxy, la chaîne hydrocarbonée dudit radical pouvant éventuellement être interrompue par au moins un atome d'oxygène ou un atome de soufre ; en outre:
  - au moins un des groupes $R^1$ à $R^8$ peut former, avec au moins un autre des groupes $R^1$ à $R^8$ et avec le ou les atomes de carbone auxquels lesdits groupes sont reliés, un cycle ou hétérocycle hydrocarboné saturé ou insaturé, éventuellement substitué, et comprenant de 3 à 10 chaînons; et
  - au moins une des paires ($R^1$, $R^2$), ($R^3$, $R^4$), ($R^5$, $R^6$) et ($R^7$, $R^8$) peut former, avec l'atome de carbone auquel ladite paire est reliée, un groupe carbonyle C=O ou un groupe de 2 atomes de carbone reliés par une double liaison : C=C, dont l'autre atome de carbone porte 2 substituants choisis parmi un atome d'hydrogène ou un radical $C_1$-$C_4$ alkyle;

- x et y sont des nombres entiers, identiques ou différents, compris dans une fourchette allant de 0 à 6, la somme x + y étant comprise dans une fourchette allant de 0 à 6 ;

- $R^9$, $R^{10}$, $R^{11}$ et $R^{12}$, identiques ou différents, représentent :

  - un atome d'hydrogène ou d'halogène ; ou
  - un radical comprenant de 1 à 22 atomes de carbone et choisi parmi alkyle, alcényle, alcoxycarbonyle, alcényloxycarbonyle, alkylcarbonyloxy, alcénylcarbonyloxy, alkylcarbonyloxyalkyl, la chaîne hydrocarbonée dudit radical pouvant éventuellement être interrompue par au moins un atome d'oxygène ou un atome de soufre ; en outre:
  - au moins un des groupes $R^9$ à $R^{12}$ peut former, avec au moins un autre des groupes $R^9$ à $R^{12}$ et avec le ou les atomes de carbone auxquels lesdits groupes sont reliés, un cycle ou hétérocycle hydrocarboné saturé ou insaturé, éventuellement substitué, et comprenant de 3 à 10 chaînons; et
  - au moins une des paires ($R^9$, $R^{10}$) et ($R^{11}$, $R^{12}$) peut former avec l'atome de carbone auquel ladite paire est reliée un groupe de 2 atomes de carbone reliés par une double liaison : C=C, dont l'autre atome de carbone porte 2 substituants choisis parmi un atome d'hydrogène ou un radical $C_1$-$C_4$ alkyle ; et
  - l'atome de carbone porteur de l'un des groupes de la paire ($R^9$, $R^{10}$) peut être relié à l'atome de carbone porteur de l'un des groupes de la paire ($R^{11}$, $R^{12}$) par une double liaison, étant entendu que, conformément aux règles de valence, un seul des groupes de chacune de ces 2 paires est alors présent ;

- $R^{13}$ représente :

  - un atome d'oxygène ou de soufre, ou
  - un radical divalent $-CH_2-$, -C(=O)- ou $-NR^0-$ dans lequel $R^0$ est un radical alkyle ou alcényle comprenant de 1 à 22 atomes de carbone ;

- n1 et n2, identiques ou différents, sont chacun un nombre entier ou égal à 0, dont la somme est désignée par n ;

- m est un nombre entier ou égal à 0 ;

- p1 et p2, identiques ou différents, sont chacun un nombre entier ou égal à 0 dont la somme p1 + p2 vérifie l'équation :
  p1 + p2 = q x (z + 1)
  dans laquelle :

- q est un nombre entier ou égal à 0 ; et
- z est un nombre entier allant de 1 à 5 ; et

- n1, n2, m, p1 et p2 étant en outre tels que la masse moléculaire moyenne en nombre Mn du polymère de formule (I) est comprise dans une fourchette allant de 400 à 100 000 g/mol et son indice de polymolécularité est compris dans un domaine allant de 1,0 à 3,0.

Les divers groupes, radicaux et lettres qui sont compris dans la formule (I) et qui sont définis ci-dessus, conservent tout au long du présent texte, et, en l'absence d'indication contraire, la même définition.

Les variantes suivantes du polymère de formule (I), prises individuellement ou en combinaison, sont particulièrement préférées :

- les radicaux $R^{14}$ et $R^{15}$ compris dans la définition de $F^1$ et $F^2$ représentent un radical $C_1$-$C_4$ alkyle, de préférence linéaire, ou bien forment avec l'atome de carbone auxquels ils sont reliés un radical cyclohexyle ;
- g et d compris dans la définition de $F^1$ et $F^2$ représentent 0 ou 1, et sont de préférence identiques ;
- $R^1$, $R^2$, $R^3$, $R^4$, $R^5$, $R^6$, $R^7$ et $R^8$ représentent un atome d'hydrogène ou un radical alkyle comprenant de 1 à 14 atomes de carbone, et encore plus préférentiellement de 1 à 8 ;
- les nombres entiers x et y sont compris dans une fourchette allant de 0 à 2, la somme x + y étant comprise dans une fourchette allant de de 0 à 2 ;
- x est égal à 1 et y est égal à 1 ;
- $R^9$, $R^{10}$, $R^{11}$ et $R^{12}$ représentent un atome d'hydrogène ou un radical dont la partie hydrocarbonée comprend de 1 à 14 atomes de carbone, et encore plus préférentiellement de 1 à 8 ;
- $R^{13}$ représente le radical divalent $-CH_2-$,
- z est un nombre entier égal à 1 ou 2 ; et/ou
- la masse moléculaire moyenne en nombre Mn est comprise dans une fourchette allant de 3000 à 50 000 g/mol, plus particulièrement de 5000 à 30 000 g/mol, et l'indice de polymolécularité est compris dans un domaine allant de 1,4 à 2,0.

La chaîne principale du polymère de formule (I) peut donc comprendre un seul motif de répétition choisi parmi :

- le motif de répétition répété p1 + p2 fois, ou
- le motif de répétition répété n1 + n2 fois ; ou encore
- le motif de répétition répété m fois.

Elle peut également comprendre 2 motifs choisis parmi deux quelconques des 3 motifs identifiés ci-dessus et elle peut également comprendre ces 3 motifs ; il est alors entendu que la répartition desdits motifs sur ladite chaîne principale est statistique, et que le polymère de formule (I) est donc un polymère statistique.

Ainsi qu'il apparaît ci-dessus, les groupements terminaux $F^1$ et $F^2$ sont généralement symétriques par rapport à la chaîne principale, c'est-à-dire qu'ils se correspondent sensiblement, à l'exception des indices g et d.

Par « groupement terminal », on entend un groupement situé à l'une des 2 extrémités de la chaîne principale du polymère, laquelle est constituée par un ou plusieurs motifs de répétition.

L'indice de polymolécularité (également dénommé en anglais polydispersity index ou PDI) est défini comme le rapport Mw / Mn, c'est-à-dire le rapport de la masse moléculaire moyenne en poids à la masse moléculaire moyenne en nombre du polymère.

Dans le présent texte, les deux masses moléculaires moyennes Mn et Mw sont mesurées par chromatographie d'exclusion stérique (ou SEC, acronyme de « Size Exclusion Chromatography » en anglais) qui est également désignée par les termes de chromatographie par perméation de gel (ou par le sigle anglais correspondant GPC). L'étalonnage mis en oeuvre est usuellement un étalonnage PEG (PolyEthylèneGlycol) ou PS (PolyStyrène), de préférence PS.

Si g = 0 ou d = 0, alors il n'y a pas de radical $-(CH_2)-$ dans les groupes $F^1$ et $F^2$ de la formule (IIa) et (IIb). En d'autres termes, le radical : $-(CH_2)_g-$ ou $-(CH_2)_d-$est remplacé par une simple liaison.

Lorsque un des indices n1, n2, p1, p2, m, x ou y qui s'applique à un ensemble de deux crochets est égal à zéro, cela signifie qu'il n'y a pas de groupe entre les crochets auxquels cet indice s'applique. Ainsi par exemple le groupement :

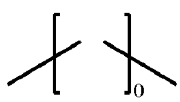

représente une simple liaison : -, et le groupement :

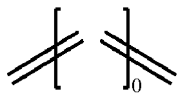

représente une double liaison : .

Les polymères de formule (I) selon l'invention sont particulièrement homogènes et stables en température.

Ils peuvent former, après une réaction de polyaddition à une température inférieure à 80°C avec une polyamine primaire et/ou secondaire un polyamide pouvant constituer un joint adhésif.

Le joint adhésif ainsi formé présente des valeurs de cohésion élevées, en particulier supérieures à 2 MPa. De telles valeurs de cohésion permettent une utilisation dudit polymère comme adhésif, par exemple comme joint d'étanchéité sur un support usuel (béton, verre, marbre), dans le domaine du bâtiment, ou encore pour le collage de vitrages dans l'industrie automobile et navale.

Les polymères de formule (I) selon l'invention sont solides ou liquides à température ambiante (i.e. environ 20°C).

Selon une variante préférée du polymère selon l'invention, lorsque m est non nul, que p1 et p2 sont non nuls et que n1 et n2 sont chacun égal à 0 (correspondant à la présence dans la chaîne principale du polymère des 2 seuls motifs de répétition répétés respectivement p1 + p2 fois et m fois), alors le rapport :

$$m / (p1 + p2 + m)$$

est compris dans l'intervalle allant de 30 à 70 %, et, de façon davantage préférée, est égal à environ 50 %.

Selon une deuxième alternative de cette même variante préférée, lorsque m est égal à 0, que p1 et p2 sont non nuls et que la somme n1 + n2 est non nulle (correspondant à la présence dans la chaîne principale du polymère des 2 seuls motifs de répétition répétés respectivement p1 + p2 fois et n1 + n2 fois), alors au moins un des groupes $R^1$, $R^2$, $R^3$, $R^4$, $R^5$, $R^6$, $R^7$ et $R^8$ est autre qu'un atome d'hydrogène, et le rapport :

$$(n1 + n2) / (p1 + p2 + n1 + n2)$$

est compris dans l'intervalle allant de 30 à 70 %, et, de façon davantage préférée, est égal à environ 50 %.

Selon une troisième alternative de cette même variante préférée, lorsque m est différent de 0, que p1 et p2 sont chacun égal à 0, que la somme n1 + n2 est non nulle (correspondant à la présence dans la chaîne principale du polymère des 2 seuls motifs de répétition répétés respectivement m fois et n1 + n2 fois), et que chacun des groupes $R^1$, $R^2$, $R^3$, $R^4$, $R^5$, $R^6$, $R^7$ et $R^8$ est un atome d'hydrogène, alors le rapport :

$$m / (m + n1 + n2)$$

est compris dans l'intervalle allant de 30 à 70 %, et, de façon davantage préférée, est égal à environ 50 %.

Selon encore une quatrième alternative de ladite variante préférée, lorsque m est non nul, que p1 et p2 sont non nuls et que la somme n1 + n2 est non nulle (correspondant à la présence dans la chaîne principale du polymère des 3 motifs de répétition) et que chacun des groupes $R^1$, $R^2$, $R^3$, $R^4$, $R^5$, $R^6$, $R^7$ et $R^8$ est un atome d'hydrogène, alors le rapport :

$$m / ( p1 + p2 + n1 + n2 + m)$$

est compris dans l'intervalle allant de 30 à 70 %, et, de façon davantage préférée, est égal à environ 50 %.

Selon encore une cinquième alternative de ladite variante préférée, lorsque m est non nul, que p1 et p2 sont non nuls et que la somme n1 + n2 est non nulle (correspondant à la présence dans la chaîne principale du polymère des 3 motifs de répétition) et que au moins un des groupes $R^1$, $R^2$, $R^3$, $R^4$, $R^5$, $R^6$, $R^7$ et $R^8$ est autre qu'un atome d'hydrogène, alors le rapport :

$$(m + n1 + n2) / ( p1 + p2 + n1 + n2 + m)$$

est compris dans l'intervalle allant de 30 à 70 %, et, de façon davantage préférée, est égal à environ 50 %.

Conformément aux 5 alternatives de ladite variante préférée, le polymère de formule (I) se présente généralement sous la forme de liquide visqueux, et est caractérisé par une viscosité Brookfield à 23°C allant de 1 mPa.s à 500 Pa.s, de préférence de 1 à 150 Pa.s et encore plus préférentiellement de 1 à 50 Pa.s. Il est alors avantageusement facile à mettre en oeuvre et peut être combiné à un constituant supplémentaire tel qu'une résine tackifiante ou une charge, pour former une composition adhésive.

Quand le polymère selon l'invention est solide à température ambiante, il est thermoplastique, c'est-à-dire déformable et fusible à chaud (i.e. à une température supérieure à la température ambiante). Il peut donc être utilisé, en mélange avec une polyamine au moment de l'emploi, en tant qu'adhésif bicomposant appliqué sur l'interface de substrats à assembler au niveau de leur surface de tangence.

Selon un mode de réalisation de l'invention, toutes les liaisons ----- de la formule (I) sont des doubles liaisons carbone-carbone, et la formule (I) devient alors la formule (I') suivante :

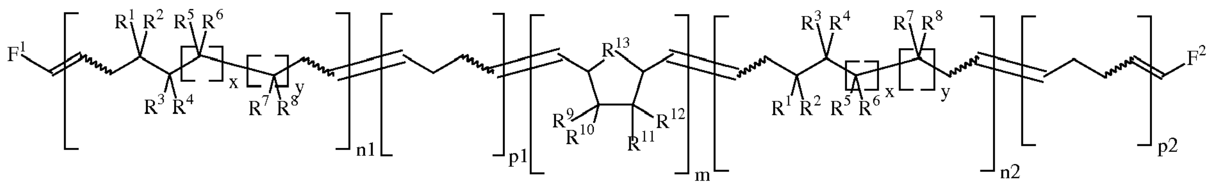

(I')

dans laquelle x, y, n1, n2, m, p1, p2, $F^1$, $F^2$, $R^1$, $R^2$, $R^3$, $R^4$, $R^5$, $R^6$, $R^7$, $R^8$, $R^9$, $R^{10}$, $R^{11}$, $R^{12}$ et $R^{13}$ ont les significations données précédemment et la liaison est une liaison orientée géométriquement d'un côté ou de l'autre par rapport à la double liaison (cis ou trans).

Chacune des doubles liaisons du polymère de formule (I') est orientée géométriquement cis ou trans, de préférence est d'orientation cis. Les isomères géométriques du polymère de formule (I') sont généralement présents en proportions variables, avec le plus souvent une majorité de doubles liaisons orientées cis (Z), et préférentiellement toutes orientées cis (Z). Il est aussi possible selon l'invention d'obtenir un seul des isomères géométriques, selon les conditions réactionnelles et en particulier selon la nature du catalyseur utilisé.

Selon un autre mode de réalisation de l'invention, toutes les liaisons ----- de la formule (I) sont des liaisons simples carbone-carbone, et la formule (I) devient alors la formule (IH) ci-dessous :

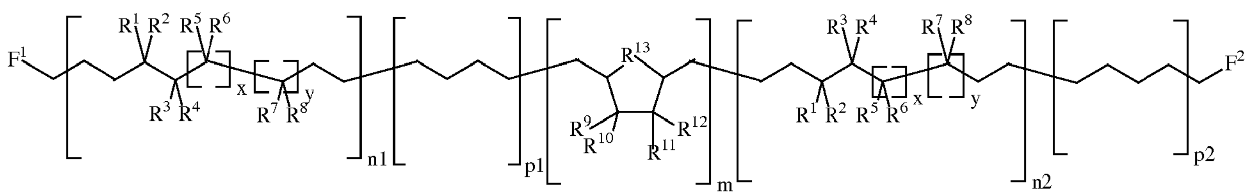

(IH)

dans laquelle x, y, n1, n2, m, p1, p2, $F^1$, $F^2$, $R^1$, $R^2$, $R^3$, $R^4$, $R^5$, $R^6$, $R^7$, $R^8$, $R^9$, $R^{10}$, $R^{11}$, $R^{12}$ et $R^{13}$ ont les significations données précédemment.

La formule (IH) illustre le cas où la chaîne principale du polymère de formule (I) est saturée, c'est-à-dire ne comporte que des liaisons saturées.

Dans ce cas, de façon préférée, x est égal à 1 et y est égal à 1.

Le polymère de formule (IH) peut par exemple être issu de l'hydrogénation du polymère insaturé de formule (I').

Selon un mode de réalisation du polymère de formule (I) selon l'invention, m, p1 et p2 sont chacun égal à 0, le polymère étant de formule (II) suivante :

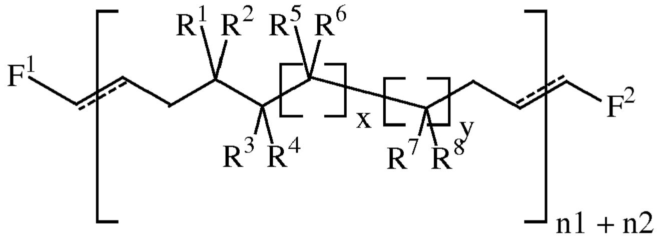

(II)

dans laquelle x, y, n1, n2, $F^1$, $F^2$, $R^1$, $R^2$, $R^3$, $R^4$, $R^5$, $R^6$, $R^7$ et $R^8$, ont les significations données précédemment.

De façon particulièrement préférée, x est égal à 1 et y est égal à 1.

Selon une forme particulièrement préférée de ce mode de réalisation, toutes les liaisons----- de la formule (II) sont des doubles liaisons carbone-carbone, et la formule (II) devient alors la formule (II') suivante :

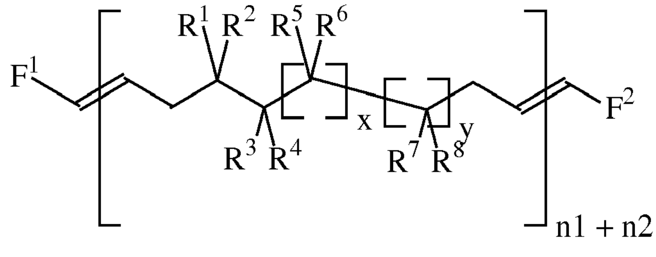

(II')

Les formules (II) et (II') illustrent le cas où la chaîne principale du polymère de formule (I) comprend un seul motif de répétition, correspondant à celui qui est répété n1 + n2 fois.

Selon un autre mode de réalisation du polymère de formule (I) selon l'invention, n1 et n2 sont chacun égal à 0, le polymère étant de formule (III) suivante :

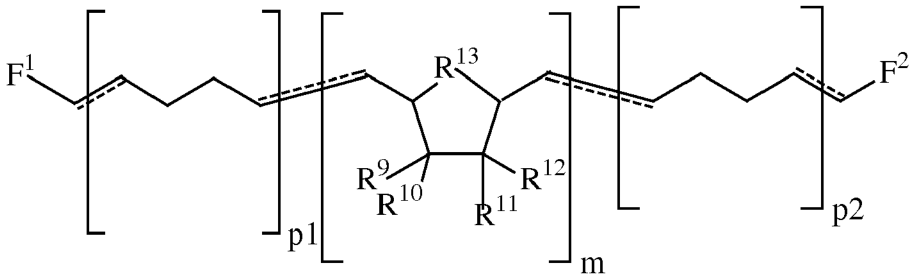

(III)

dans laquelle m, p1, p2 $F^1$, $F^2$, $R^9$, $R^{10}$, $R^{11}$, $R^{12}$ et $R^{13}$ ont les significations données précédemment.

Selon une forme particulièrement préférée de ce mode de réalisation, toutes les liaisons ----- de la formule (III) sont des doubles liaisons carbone-carbone, et la formule (III) devient alors la formule (III') suivante :

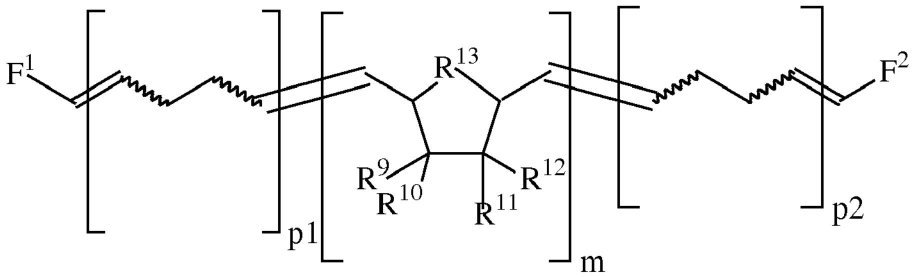

(III')

Les formules (III) et (III') illustrent le cas où la chaîne principale du polymère de formule (I) comprend deux motifs de répétition, correspondant à ceux qui sont répétés respectivement (p1 + p2) fois et m fois.

S'agissant des groupements terminaux azlactones du polymère selon l'invention, de préférence $R^{14}$ et $R^{15}$ représentent chacun un radical méthyle. Dans ce cas, $F^1$ et $F^2$ sont identiques et représentent ou bien le radical :

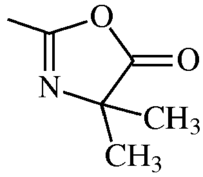

ou bien le radical :

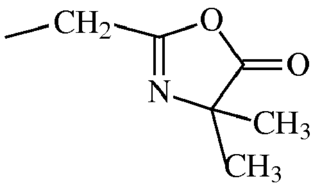

Selon une autre variante préférée, $R^{14}$ et $R^{15}$ forment avec l'atome de carbone auxquels ils sont reliés un cyclohexyle. Dans ce cas, $F^1$ et $F^2$ sont identiques et représentent le radical :

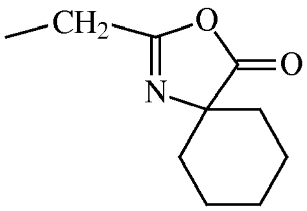

L'invention concerne encore un procédé de préparation d'un polymère hydrocarboné comprenant deux groupements terminaux azlactones de formule (I) selon l'invention, ledit procédé comprenant au moins une réaction de polymérisation par ouverture de cycle par métathèse (également dénommée « Ring-Opening Metathesis Polymerization» ou ROMP en anglais), en présence :

(a) d'un catalyseur de métathèse ;

(b) d'un agent de transfert de chaîne (désigné également ci-après par CTA pour « Chain Transfer Agent » en anglais) comprenant 2 groupements azlactones, de formule (B) suivante :

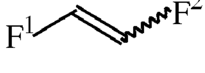

(B)

dans laquelle :

- $F^1$ et $F^2$ sont tels que définis précédemment ;
- la liaison est une liaison orientée géométriquement d'un côté ou de l'autre par rapport à la double liaison (cis ou trans) ; et

(c) d'au moins un composé C choisi parmi :

- le composé de formule (C1) :

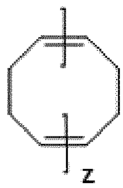

(C1)

dans laquelle z est tel que défini précédemment ;
- le composé de formule (C2) :

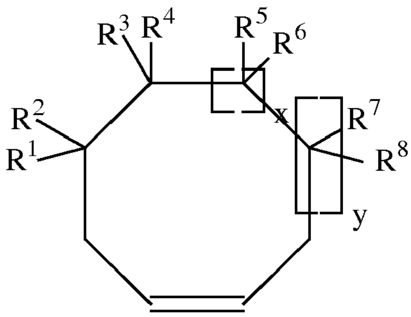

(C2)

dans laquelle $R^1$, $R^2$, $R^3$, $R^4$, $R^5$, $R^6$, $R^7$, $R^8$, x et y sont tels que définis précédemment ; et
- le composé de formule (C3) :

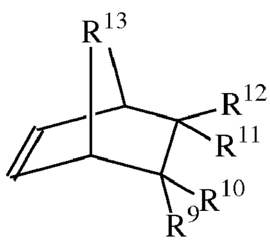

(C3)

dans laquelle $R^9$, $R^{10}$, $R^{11}$, $R^{12}$ et $R^{13}$ sont tels que définis précédemment ;
ladite réaction de polymérisation étant en outre mise en oeuvre :

- pendant une durée allant de 2 à 24 heures et à une température comprise dans un intervalle de 20 à 70 °C ; et
- avec un rapport r, égal au rapport du nombre de moles dudit CTA au nombre total de moles du composé C, compris dans un intervalle allant de 0,0010 à 1,0.

Dans la définition du procédé de préparation donnée ci-dessus, il est bien entendu que l'article indéfini "un", en tant qu'il se rapporte à un réactif ou au catalyseur mis en oeuvre doit s'interpréter comme signifiant "au moins un", c'est-à-dire "un ou plusieurs". Notamment ledit procédé peut mettre en oeuvre un seul ou plusieurs composé(s) C ayant ou bien la même formule ou bien une formule différente, de préférence de formule différente. Lorsque le procédé met

en oeuvre plusieurs composé(s) C, le dénominateur du rapport r défini précédemment est la somme du nombre total de moles des composés C mis en oeuvre.

Selon une variante préférée dudit procédé, un seul composé C est mis en oeuvre, répondant à la formule (C2).

Selon une autre variante préférée dudit procédés, deux composés C sont mis en oeuvre, répondant l'un à la formule (C1) et l'autre à la formule (C3).

La durée et la température de la réaction dépendent généralement de ses conditions de mise en oeuvre notamment de la nature du solvant utilisé, et en particulier du taux de charge catalytique. L'homme du métier est à même de les adapter en fonction des circonstances.

Ainsi, de préférence, la durée de la réaction de polymérisation va de 2 à 10 heures, et le rapport r défini ci-dessus est compris dans un intervalle allant de 0,0010 à 0,3.

**(a) Catalyseur de métathèse :**

Le catalyseur de métathèse est, de préférence, un catalyseur comprenant du ruthénium, et de façon encore plus préférée un catalyseur de Grubbs,

Un tel catalyseur est généralement un produit du commerce.

Le catalyseur de métathèse est le plus souvent un catalyseur de métal de transition dont notamment un catalyseur comprenant du ruthénium le plus souvent sous forme de complexe(s) du ruthénium tel qu'un complexe ruthénium-carbène.

Par catalyseur de Grubbs, on entend généralement selon l'invention un catalyseur de Grubbs de 1ère ou 2ème génération, mais aussi tout autre catalyseur de type Grubbs (de type ruthénium-carbène) ou Hoveyda-Grubbs accessible à l'homme du métier, tel que par exemple les catalyseurs de Grubbs substitués décrits dans le brevet US 5,849,851.

Un catalyseur de Grubbs 1ère génération est généralement de formule (G1) :

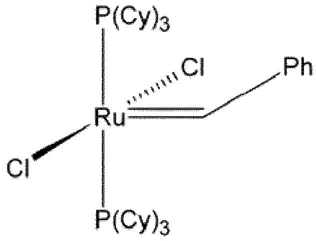

(G1)

dans laquelle Ph est le phényle, Cy est le cyclohexyle, et le groupe $P(Cy)_3$ est un groupe tricyclohexylphosphine. La dénomination IUPAC de ce composé est : benzylidène-bis(tricyclohexylphosphine) dichlororuthénium (de numéro CAS 172222-30-9). Un tel catalyseur est disponible notamment auprès de la société Aldrich.

Un catalyseur de Grubbs 2ème génération (ou G2) est généralement de formule (G2) :

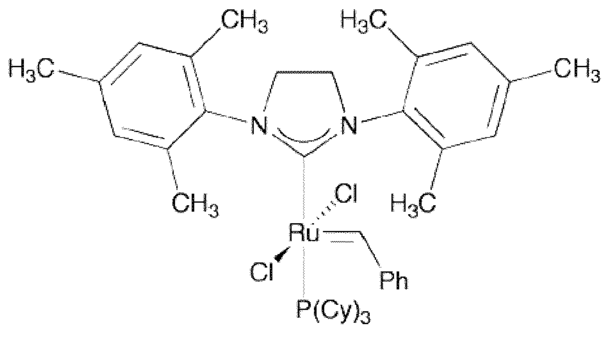

(G2)

dans laquelle Ph est le phényle et Cy est le cyclohexyle.
La dénomination IUPAC de la deuxième génération de ce catalyseur est benzylidène [1,3-bis(2,4,6-triméthylphényl)-2-imidazolidinylidène] dichloro(tricyclohexylphosphine) ruthénium (de numéro CAS 246047-72-3). Ce catalyseur est également disponible auprès de la société Aldrich.

**(b) CTA de formule (B)** :

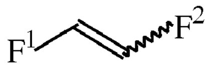

(B)

Le CTA de formule (B) peut être préparé à partir d'acides dicarboxyliques insaturés comprenant de 4 à 18 atomes de carbone et à partir d'un α-aminoacide naturel ou synthétique selon le mode opératoire décrit ci-dessous :

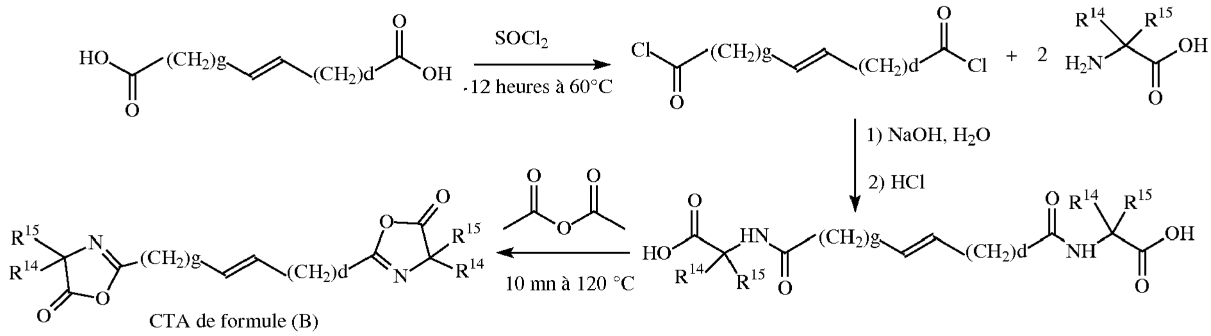


On peut citer comme exemples d' α-aminoacide naturel susceptible d'être mis en oeuvre dans ce mode opératoire : l'alanine, la valine, l'isoleucine, la leucine, la méthionine ou encore la phénylalanine.

Les α-aminoacides synthétiques monosubstitués en α et les α-aminoacides synthétiques bisubstitués en α peuvent être préparés selon Ooi, T. ; Maruoka, K. Angew. Chem. Int. Ed. 2007, 46, 4222 et Ooi, T. ; Maruoka, K. Aldrichmica Acta 2007, 40,77.

L' α-aminoacide dans lequel $R^{14}$ et $R^{15}$ forment avec l'atome de carbone auxquels ils sont reliés un radical cyclohexyle peut être préparé à partir de cyclohexanone selon le mode opératoire décrit dans Aboul-Enein, M.N. ; El-Azzouny, A. A. ; Abdallah, N. A. ; Makhlouf, A. A. Egyptian Journal of Chemistry, 1991, vol. 34, # 6 p.549-558.

Conformément à une variante préférée de l'invention, les groupes $R^{14}$ et $R^{15}$ compris dans la formule (B) du CTA représentent chacun un radical méthyle. Dans ce cas, $F^1$ et $F^2$ sont identiques et le CTA de formule (B) est avantageusement :

- le composé de formule :

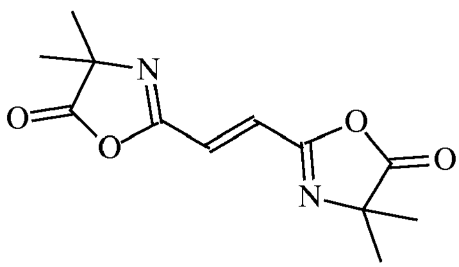

dénommé ci-après CTA $AzI_2$ ; ou bien

- le composé de formule :

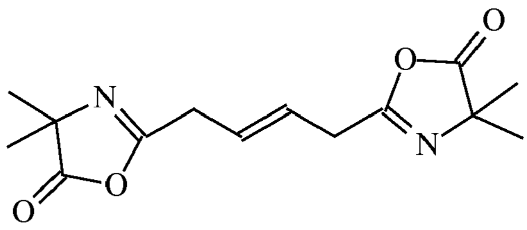

dénommé ci-après CTA di-$CH_2$-Azl.

Conformément à une autre variante préférée de l'invention, les groupes $R^{14}$ et $R^{15}$ forment avec l'atome de carbone auxquels ils sont reliés un cyclohexyle. Dans ce cas, $F^1$ et $F^2$ sont identiques et le CTA de formule (B) est avantageusement le composé de formule :

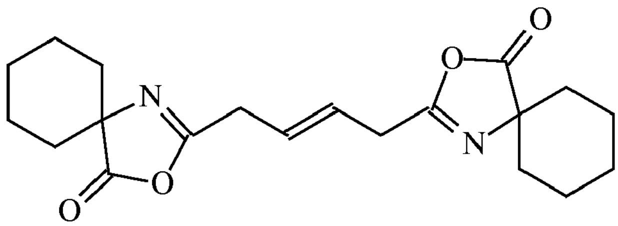

dénommé ci-après CTA di-$CH_2$-HexAzl.

**<u>(c) composé C de formule (C1)</u> :**

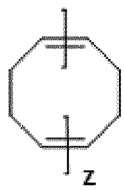

(C1)

Le composé cyclique de formule (C1) comprend généralement de 8 à 32 atomes de carbones.
De préférence, il est choisi dans le groupe formé par :

- le 1,5-cyclooctadiène (désigné ci-après par COD) de formule :

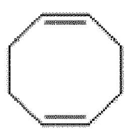

(correspondant à z =1)

- et le 1,5,9-cyclododecatriène (désigné ci-après par CDT) composé à 12 atomes de carbone de formule :

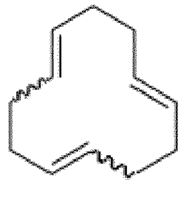

(correspondant à z =2)

ces 2 composés étant disponibles commercialement auprès des sociétés Evonik Degussa et Arkema France.

**(d) composé C de formule (C2) :**

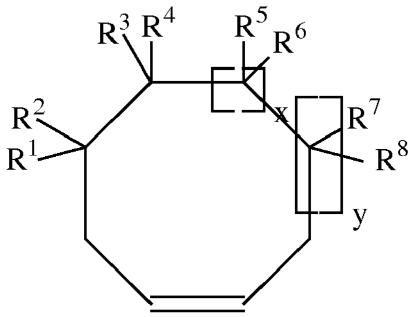

(C2)

Le composé de formule (C2) comprend généralement de 6 à 30, de préférence de 6 à 22, atomes de carbone.
De préférence :

- $R^1$, $R^2$, $R^3$, $R^4$, $R^5$, $R^6$, $R^7$ et $R^8$ représentent un atome d'hydrogène ou un radical alkyle comprenant de 1 à 14 atomes de carbone, et encore plus préférentiellement de 1 à 8 ;
- les nombres entiers x et y sont compris dans une fourchette allant de 0 à 2, la somme x + y étant comprise dans une fourchette allant de de 0 à 2.

Selon une variante encore davantage préférée :

- x est égal à 1 et y est égal à 1 et/ou
- au plus un des groupes pris parmi ($R^1$, $R^2$, $R^3$, $R^4$, $R^5$, $R^6$, $R^7$ et $R^8$) est un radical $C_1$-$C_8$ alkyle et tous les autres représentent un atome d'hydrogène.

Le composé de formule (C2) est notamment choisi parmi :

- le cycloheptène, le cyclooctène, le cyclononène, le cyclodécène, le cycloundécène et le cyclododécène.
- le 5-époxy-cyclooctène, de formule :

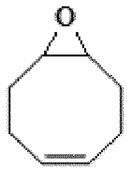

(disponible chez Aldrich),
- le 5-oxocyclooctène, de formule :

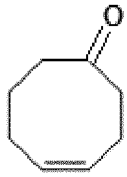

ou encore parmi un 5-alkyl-cyclooctène, de formule :

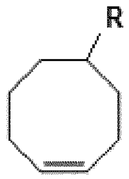

dans laquelle R est un radical alkyle comprenant de 1 à 22 atomes de carbone, de préférence 1 à 14 ; R étant par exemple le radical n-hexyle.

Parmi ces composés, est tout particulièrement préféré le cyclooctène.

**<u>(e) composé C de formule (C3)</u> :**

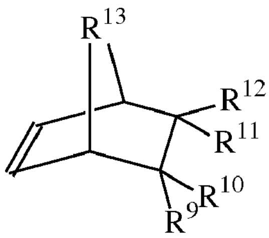

(C3)

Le composé de formule (C3) comprend généralement de 6 à 30, de préférence de 6 à 22, atomes de carbone.
De préférence :

- $R^9$, $R^{10}$, $R^{11}$ et $R^{12}$ représentent un atome d'hydrogène ou un radical alkyle ou alkoxycarbonyle comprenant de 1 à 14 atomes de carbone, et encore plus préférentiellement de 1 à 8 ;
- le radical $R^0$, compris dans le groupe $-NR^0$ qui est l'une des significations de $R^{13}$, est un radical linéaire comprenant de 1 à 14 atomes de carbone.

Selon une variante encore davantage préférée :

- au plus un des groupes pris parmi ($R^9$, $R^{10}$, $R^{11}$ et $R^{12}$) est un radical $C_1$-$C_8$ alkoxycarbonyle et tous les autres représentent un atome d'hydrogène ; et/ou
- $R^{13}$, représente un radical $-CH_2-$ ou un atome d'oxygène.

Le composé de formule (E) est notamment choisi parmi :

- le norbornène, de formule suivante :

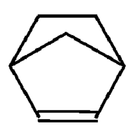

- le norbornadiène, de formule suivante:

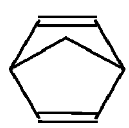

- le dicyclopentadiène, de formule suivante:

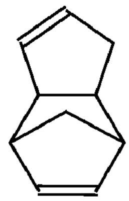

- le 7-oxanorbornène, de formule suivante:

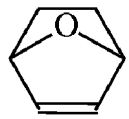

- le 7-oxanorbornadiène, de formule suivante:

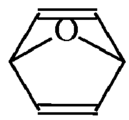

- le 5-éthylidène-2-norbornène, de formule suivante:

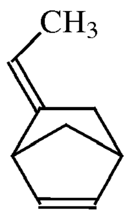

- ou encore, le 5-norbonene-2-methylacetate, de formule suivante:

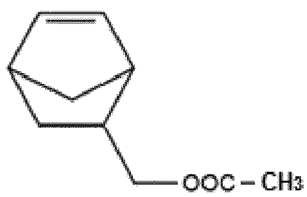

Le composé de formule (C3) peut également être choisi parmi les composés de formules suivantes :

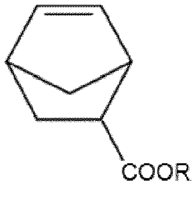 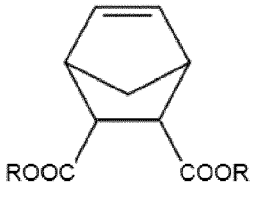 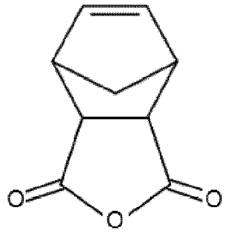 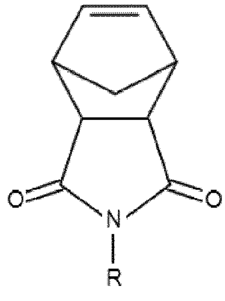

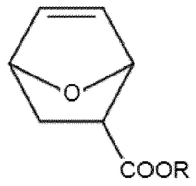
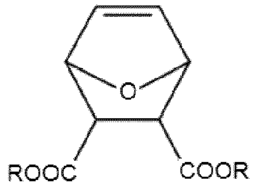
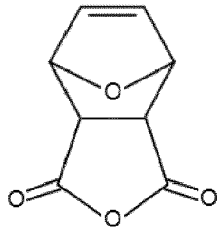
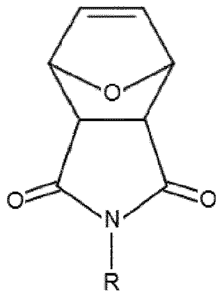

dans lesquelles R est tel que défini précédemment pour le composé de formule (C2).

Le composé de formule (C3) peut également être choisi dans le groupe formé par les produits d'addition (ou adducts en anglais) issus de la réaction de Diels-Alder utilisant le cyclopentadiène ou le furane comme produit de départ, ainsi que les composés dérivés du norbornène tels les norbornènes branchés tels que décrits dans WO 2001/04173 (tels que : carboxylate d'isobornyl norbornène, carboxylate de phényl norbornène, carboxylate d'éthylhéxyle norbornène, carboxylate de phénoxyéthyle norbornène et alkyl dicarboxymide norbornène, l'alkyl comportant le plus souvent de 3 à 8 atomes de carbone) et les norbornènes branchés tels que décrits dans WO 2011/038057 (anhydrides dicarboxyliques de norbornène et éventuellement anhydrides dicarboxyliques de 7-oxanorbornène).

Parmi les différents composés de formule (C3) cités, sont tout particulièrement préférés le norbornène, le 7-oxanorbornène, le 5-norbornene-2-carboxylate de méthyle, de formule :

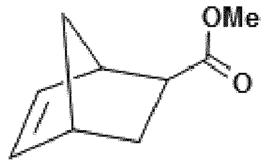

le 5-oxanorbornene-2-carboxylate de méthyle, de formule :

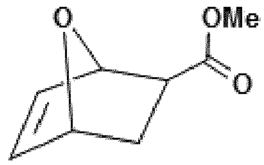

ou encore le dicyclopentadiène.

L'étape de polymérisation par ouverture de cycle par métathèse (ou ROMP pour « Ring-Opening Metathesis Polymerization » en anglais) est mise en oeuvre le plus souvent en présence d'au moins un solvant, généralement choisi dans le groupe formé par les solvants aqueux ou organiques typiquement utilisés dans les réactions de polymérisation et qui sont inertes dans les conditions de la polymérisation, tels que les hydrocarbures aromatiques, les hydrocarbures chlorés, les éthers, les hydrocarbures aliphatiques, les alcools, l'eau ou leurs mélanges. Un solvant préféré est choisi dans le groupe formé par le benzène, le toluène, le para-xylène, le chlorure de méthylène, le dichloroéthane, le dichlorobenzène, le chlorobenzène, le tétrahydrofurane, le diéthyl éther, le pentane, l'hexane, l'heptane, un mélange d'isoparaffines liquides (par exemple l'isopar®), le méthanol, l'éthanol, l'eau ou leurs mélanges. De façon encore plus préférée, le solvant est choisi dans le groupe formé par le benzène, le toluène, le paraxylène, le chlorure de méthylène, le 1,2-dichloroéthane, le dichlorobenzène, le chlorobenzène, le tétrahydrofurane, le diéthyl éther, le pentane, l'hexane, l'heptane, le méthanol, l'éthanol ou leurs mélanges. De façon encore plus particulièrement préférée, le solvant est le dichlorométhane, le 1,2-dicholoroéthane, le toluène, l'heptane, ou un mélange de toluène et de 1,2-dichloroéthane. La solubilité du polymère formé au cours de la réaction de polymérisation dépend généralement et principalement du choix du solvant, de la nature et de la proportion des comonomères et de la masse moléculaire moyenne en nombre du polymère obtenu. Il est aussi possible que la réaction soit mise en oeuvre sans solvant.

Le procédé de préparation d'un polymère hydrocarboné selon l'invention peut comporter en outre au moins une étape supplémentaire d'hydrogénation de doubles liaisons.

Cette étape est généralement mise en oeuvre par hydrogénation catalytique, le plus souvent sous pression d'hydrogène et en présence d'un catalyseur d'hydrogénation tel qu'un catalyseur de palladium supporté par du carbone

(Pd/C). Elle permet plus particulièrement de former un composé saturé de formule (IH) à partir d'un composé insaturé de formule (I'), et notamment les composés saturés correspondant aux composé de formule (II') et (III') à partir des composés insaturés.

L'invention concerne également l'utilisation comme adhésif du polymère hydrocarboné comprenant deux groupements terminaux azlactones, tel que défini précédemment, en mélange avec un composé aminé comprenant au moins deux groupements amines, par exemple choisi parmi les diamines, les triamines et homologues supérieurs. Les quantités du polymère hydrocarboné et du composé aminé correspondent à des quantités stoechiométriques, c'est-à-dire que le rapport molaire du nombre de groupes azlactones sur le nombre de groupes amines va de 0,8 à 1,2, de préférence de 0,9 à 1,1, voire est d'environ 1,0.

En pratique le polymère hydrocarboné et le composé aminé, utilisé comme durcisseur, sont avantageusement compris chacun dans un composant d'une composition bi-composants qui est mise à la disposition de l'utilisateur. Ce dernier procède ainsi, au moment de l'emploi de l'adhésif, au mélange de ces 2 composants, éventuellement à chaud, de manière à obtenir une composition adhésive liquide.

L'invention concerne encore un procédé d'assemblage de deux substrats par collage, comprenant :

- l'enduction sur au moins un des deux substrats à assembler d'une composition adhésive liquide obtenue par mélange d'un composé aminé comprenant au moins deux groupements amines avec le polymère hydrocarboné comprenant deux groupements terminaux azlactones tel que défini précédemment ; puis
- la mise en contact effective des deux substrats.

La composition adhésive liquide est soit la composition adhésive comprenant lesdits composé et polymère à l'état liquide à température ambiante, soit la composition adhésive fondue à chaud. L'homme du métier est à même de procéder de façon à ce que la composition adhésive utilisée soit sous forme liquide au moment de son utilisation.

L'enduction de la composition adhésive liquide est, de préférence, réalisée sous la forme d'une couche d'épaisseur comprise dans une fourchette de 0,3 à 5 mm, de préférence de 1 à 3 mm, sur au moins l'une des deux surfaces qui appartiennent respectivement aux deux substrats à assembler, et qui sont destinées à être mises en contact l'une avec l'autre selon une surface de tangence. La mise en contact effective des deux substrats est alors mise en oeuvre selon leur surface de tangence.

Bien entendu, l'enduction et la mise en contact doivent être effectuées dans un intervalle de temps compatible, comme il est bien connu de l'homme du métier, c'est-à-dire avant que la couche d'adhésif appliquée sur le substrat ne perde sa capacité à fixer par collage ce substrat à un autre substrat. En général, la polycondensation du polymère hydrocarboné avec le composé aminé commence à se produire lors de l'enduction, puis continue à se produire lors de l'étape de mise en contact des deux substrats.

Les substrats appropriés sont, par exemple, des substrats inorganiques tels que le verre, les céramiques, le béton, les métaux ou les alliages (comme les alliages d'aluminium, l'acier, les métaux non-ferreux et les métaux galvanisés) ; ou bien des substrats organiques comme le bois, des plastiques comme le PVC, le polycarbonate, le PMMA, le polyéthylène, le polypropylène, les polyesters, les résines époxy ; les substrats en métal et les composites revêtus de peinture (comme dans le domaine automobile).

Les exemples suivants sont donnés à titre purement illustratif de l'invention, et ne sauraient être interprétés pour en limiter la portée.

Les exemples 1 à 5 décrivent la préparation de polymères comprenant 2 groupements terminaux azlactones, au moyen d'une polymérisation par ouverture de cycle par métathèse.

**Exemple 1 :** polymérisation du cyclooctène (composé C de formule (C2)) en présence du CTA $AzI_2$ :

On utilise du cyclooctène (dénommé ci-après COE) disponible commercialement et le CTA $AzI_2$ de formule :

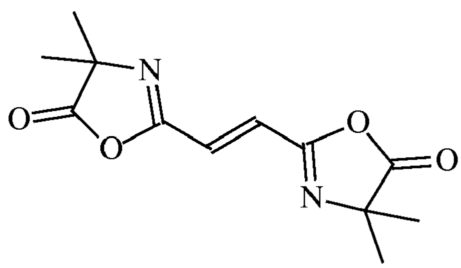

Le COE (10,8 mmol) et du 1,2-dichloroéthane sec (5 ml) sont introduits dans un ballon de 20 ml dans lequel

a également été placé un barreau magnétique d'agitation revêtu de Téflon®. Le ballon et son contenu sont ensuite mis sous argon.

Le composé CTA $AzI_2$ (0,216 mmol) est ensuite ajouté sous agitation dans le ballon par seringue. Le rapport des réactifs exprimé en nombre de moles : CTA $AzI_2$/COE est de 0,020.

Le ballon est alors immergé dans un bain d'huile à 60°C, puis l'on procède immédiatement à l'ajout, par une canule, du catalyseur G2 défini précédemment (5,4 μmol) en solution dans du 1,2-dichloroéthane (2 ml).

Le mélange réactionnel devient très visqueux en l'espace de 10 minutes. La viscosité décroit ensuite lentement pendant les heures suivantes.

Au bout de 8 heures à compter de l'addition du catalyseur, le produit présent dans le ballon est extrait après évaporation du solvant sous vide. Le produit est alors récupéré sous la forme de poudre solide incolore, après précipitation dans le méthanol, filtration et séchage à 20 °C sous vide, avec un taux de conversion du CTA de 50%.

L'analyse RMN $^1H/^{13}C$ du polymère obtenu donne les valeurs suivantes :

RMN 1 H (CDCl3, 400 MHz, 298 K) δ (ppm) = - unité de répétition : 1.29 (8H*n), 1.96 (4H*n), 5.39 (2H*n); groupement terminal : 1.53 OC(O)C(C*H3*)2NCCH, 5.30 OC(O)C(CH3)2NCC*H*CH, 5.79 OC(O)C(CH3)2NCCHC*H*.

RMN 13C (CDCl3, 100 MHz, 298 K) δ (ppm) = -unité de répétition : 29.8 (4C*n), 33.7 (2C*n), 130.1 (2C*n); groupement terminal : 24.3 OC(O)C(*C*H3)2NCCH, 70.4 OC(O)*C*(CH3)2NCCH, 121.3 OC(O)C(CH3)2NC*C*H, 161.4 OC(O)C(CH3)2N*C*CH, 181.1 O*C*(O)C(CH3)2NCCH;

Ces valeurs confirment la structure suivante :

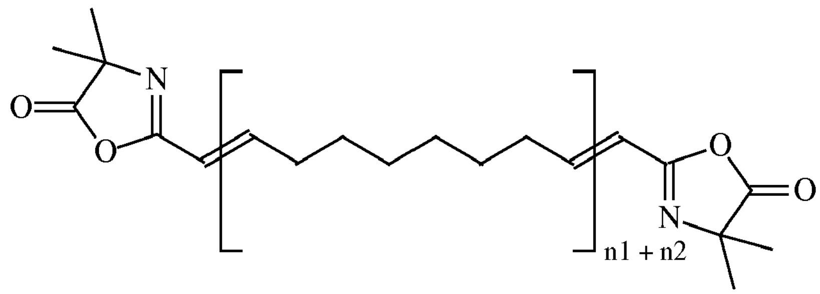

Cette structure est bien couverte par la formule (II') définie précédemment.

La masse moléculaire moyenne en nombre Mn, mesurée par RMN est de 6900 g/mol.

L'indice de polymolécularité égal au rapport Mw/Mn (mesuré par chromatographie d'exclusion stérique avec étalon de polystyrène) est de 1,4.

<u>**Exemple 2**</u> **:** polymérisation du COE en présence du CTA di-$CH_2$-AzI :

On répète l'exemple 1 en remplaçant le CTA $AzI_2$ par le CTA di-$CH_2$-AzI de formule:

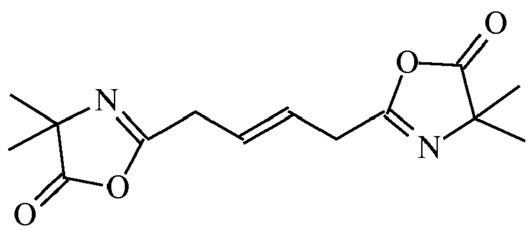

On récupère également un polymère sous la forme de poudre solide incolore, mais avec un taux de conversion du CTA de 100%, dont l'analyse RMN $^1H/^{13}C$ donne les valeurs suivantes :

RMN 1 H (CDCl3, 400 MHz, 298 K) δ (ppm) = - unité de répétition : 1.29 (8H*n), 1.96 (4H*n), 5.39 (2H*n); groupement terminal : 1.42 (s, 12H) OC(O)C(C*H3*)2NCCH2CH, 3.17 (m, 4H) OC(O)C(CH3)2NCC*H2*CH.

RMN 13C (CDCl3, 100 MHz, 298 K) δ (ppm) = -unité de répétition : 29.8 (4C*n), 33.7 (2C*n), 130.1 (2C*n); groupement terminal : 25.0 OC(O)C(*C*H3)2NCCH2CH, 40.6 OC(O)C(CH3)2NC*C*H2CH, 57.4

OC(O)*C*(CH3)2NCCH2CH, 131.2 OC(O)C(CH3)2NCCH2*C*H, 173.1 OC(O)C(CH3)2N*C*CH2CH, 176.0 O*C*(O)C(CH3)2NCCH2CH;

Ces valeurs confirment la structure ci-dessous, également couverte par la formule (II') :

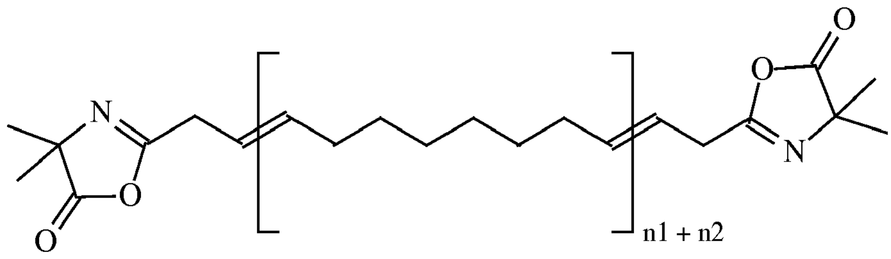

La masse moléculaire moyenne en nombre Mn et l'indice de polydispersité sont, respectivement, de 6000 g/mol et de 1,3.

__Exemple 3__ : polymérisation du 1,5,9-cyclododécatriène (composé C de formule (C1)) et du norbornène (composé C de formule (C3)) en présence du CTA di-$CH_2$-Azl:

On répète l'exemple 2 en remplaçant les 10,8 mmol de COE par un mélange de 5,4 mmol de 1,5,9-cyclododécatriène (dénommé également CDT) et de 5,4 mmol de norbornène, de formule :

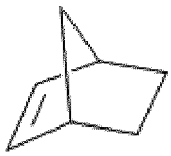

et disponible auprès de la société Sigma Aldrich.

Le rapport des réactifs exprimé en nombre de moles : CTA di-$CH_2$-Azl /(CDT + norbornène) est de 0,020.

On récupère également un polymère sous la forme d'un liquide visqueux incolore, avec un taux de conversion du CTA de 100%, dont l'analyse RMN $^1H/^{13}C$ donne les valeurs suivantes :

1 H NMR: δ (ppm) unité de répétition trans : 1.08 (2H*n), 1.39 (4H*n), 2.07 (4H*n), 2.47 (2H*n trans), 5.24-5.44 (4H*n trans), cis : 1.82-1.91(6H*n), 2.07 (4H*n), 2.82 (2H*n cis), 5.24-5,44 (4H*n cis), groupement terminal = 1.42 (s, 12H) OC(O)C(C*H3*)2NCCH2CH, 3.17 (m, 4H) OC(O)C(CH3)2NC*CH2*CH.

13C NMR: δ (ppm) unité de répétition: 27.4, 33.1, 42.1, 43.4, 130.3, 133.1, groupement terminal = 25.0 OC(O)C(*C*H3)2NCCH2CH, 40.6 OC(O)C(CH3)2NC*C*H2CH, 57.4 OC(O)*C*(CH3)2NCCH2CH, 131.2 OC(O)C(CH3)2NCCH2*C*H, 173.1 OC(O)C(CH3)2N*C*CH2CH, 176.0 O*C*(O)C(CH3)2NCCH2CH.

Ces valeurs confirment la structure ci-dessous :

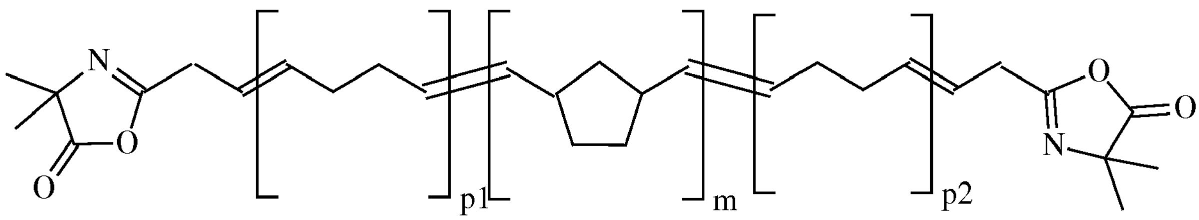

Cette structure est bien couverte par la formule (III') définie précédemment.

La masse moléculaire moyenne en nombre Mn et l'indice de polydispersité sont, respectivement, de 6900 g/mol et de 1,5.

__Exemple 4__ : polymérisation du COE en présence du CTA di-$CH_2$-HexAzl :

On répète l'exemple 1 en remplaçant le CTA $Azl_2$ par le CTA di-$CH_2$-HexAzl, de formule :

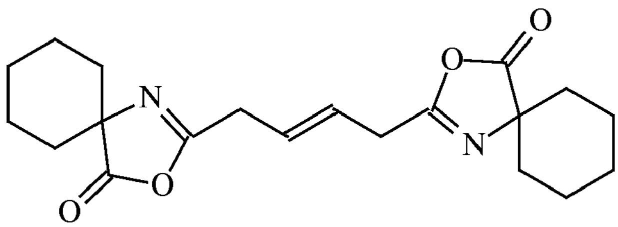

On récupère également un polymère sous la forme de poudre solide incolore, mais avec un taux de conversion du CTA de 100%, dont l'analyse RMN $^{1}H/^{13}C$ donne les valeurs suivantes :

RMN 1 H (CDCl3, 400 MHz, 298 K) δ (ppm) = - unité de répétition : 1.29 (8H*n), 1.96 (4H*n), 5.39 (2H*n); groupement terminal 1.42-1.62 (m, 20H) OC(O)C(C5*H10*)NCCH2CH, 3.25 (m, 4H) OC(O)C(C5H10)NCC*H2*CH, 5.65 (s, 2H) OC(O)C(C5H10)NCCH2C*H*;
RMN 13C (CDCl3, 100 MHz, 298 K) δ (ppm) = -unité de répétition : 29.8 (4C*n), 33.7 (2C*n), 130.1 (2C*n); groupement terminal : 20.7; 24.45; 32.8 OC(O)C(*C5*H10)NCCH2CH, 29.9 OC(O)C(C5H10)NC*C*H2CH, 68.2 OC(O)*C*(C5H10)NCCH2CH, 120.5 OC(O)C(C5H10)NCCH2*C*H, 162.4 OC(O)C(C5H10)N*C*CH2CH, 182.6 O*C*(O)C(C5H10)NCCH2CH;

Ces valeurs confirment la structure ci-dessous :

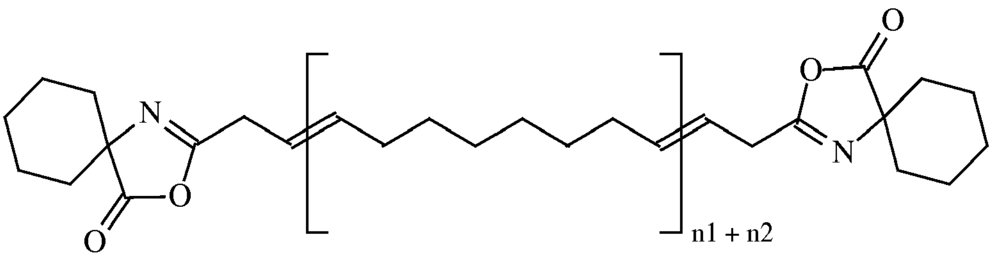

Cette structure est bien couverte par la formule (II') définie précédemment.
La masse moléculaire moyenne en nombre Mn et l'indice de polydispersité sont, respectivement, de 5500 g/mol et de 1,3.

__Exemple 5__ : polymérisation du CDT et du norbornène en présence du CTA di-$CH_2$-HexAzl :

On répète l'exemple 3 en remplaçant le CTA di-$CH_2$-Azl par le CTA di-$CH_2$-HexAzl.
On obtient également un copolymère sous la forme d'un liquide visqueux incolore, avec un taux de conversion du CTA de 100%, dont l'analyse RMN 1 H/13C donne les valeurs suivantes :

1 H NMR: δ (ppm) unité de répétition trans : 1.08 (2H*n), 1.39 (4H*n), 2.07 (4H*n), 2.47 (2H*n trans), 5.24-5.44 (4H*n trans), cis : 1.82□.91 (6H*n), 2.07 (4H*n), 2.82 (2H*n cis), 5.24-5,44 (4H*n cis), groupement terminal = 1.42-1.62 (m, 20H) OC(O)C(C5*H10*)NCCH2CH, 3.25 (m, 4H) OC(O)C(C5H10)NCC*H2*CH;
13C NMR: δ (ppm) unité de répétition: 27.4, 33.1, 42.1, 43.4, 130.3, 133.1, groupement terminal = 20.7; 24.45; 32.8 OC(O)C(*C5*H10)NCCH2CH, 29.9 OC(O)C(C5H10)NC*C*H2CH, 68.3 OC(O)*C*(C5H10)NCCH2CH, 131.7 OC(O)C(C5H10)NCCH2*C*H, 162.2 OC(O)C(C5H10)N*C*CH2CH, 181.0 O*C*(O)C(C5H10) NCCH2CH;

Ces valeurs confirment la structure :

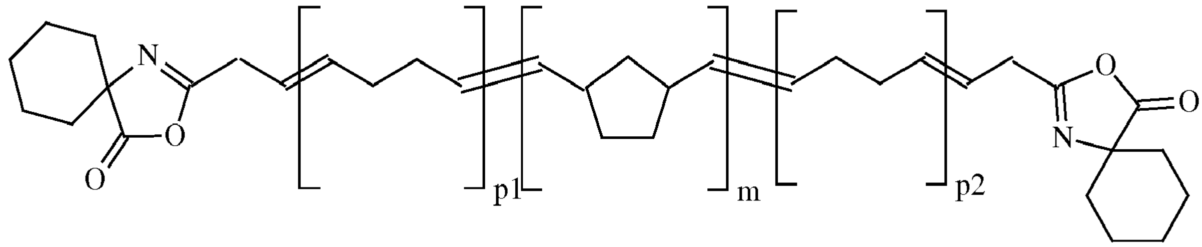

Cette structure est bien couverte par la formule (III') définie précédemment.

La masse moléculaire moyenne en nombre Mn et l'indice de polydispersité sont, respectivement, de 6600 g/mol et de 1,6.

<u>**Exemple 6**</u> : Synthèse de polyamide à partir de la polyoléfine insaturée comprenant deux groupements terminaux azlactones de l'exemple 5

Un mélange stoechiométrique dans la méthyl éthyl cétone de la polyoléfine comprenant deux groupements azlactones obtenue à l'exemple 5 avec une diamine primaire de type polyéther diamine (JEFFAMINE EDR 176, Huntsman) a été laissé à température ambiante, et ce, jusqu'à disparition complète de la bande infrarouge caractéristique des groupements azlactones (à 1815 $cm^{-1}$) et apparition des bandes caractéristiques de la liaison amide (bandes à 1550 et 1530 $cm^{-1}$). La durée de la réaction a été d'environ 1 heure.

Le produit ainsi synthétisé a conduit à la formation de polyamide, lequel formulé de façon adéquate a permis d'obtenir des propriétés adhésives.

## Revendications

1. Polymère hydrocarboné comprenant deux groupements terminaux azlactones, ledit polymère hydrocarboné ayant la formule (I) :

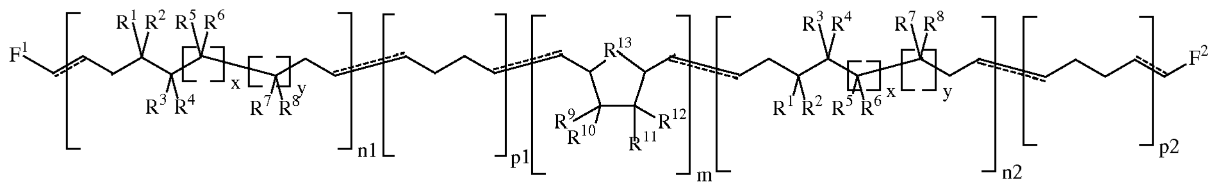

(I)

dans laquelle :

- $F^1$ représente un radical de formule (IIa) et $F^2$ représente un radical de formule (IIb):

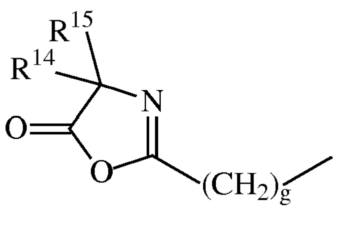

(IIa)

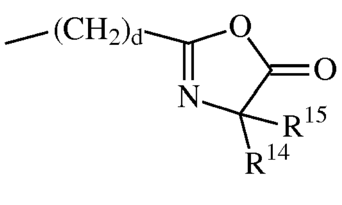

(IIb)

dans lesquelles :

- g et d, identiques ou différents, représentent un nombre entier égal à 0, 1, 2 ou 3 ;
- $R^{14}$ et $R^{15}$, identiques ou différents, représentent un atome d'hydrogène ou un groupement hydrocarboné aromatique ou aliphatique, linéaire ou ramifié, comprenant de 1 à 20 atomes de carbone pouvant être interrompu par un ou plusieurs atome d'oxygène ou de soufre ; en outre les groupes $R^{14}$ et $R^{15}$ peuvent

former avec l'atome de carbone auxquels ils sont reliés un cycle hydrocarboné comprenant de 4 à 10 chaînons et éventuellement un ou plusieurs hétéroatomes choisis parmi l'oxygène et le soufre ;

- chaque liaison carbone - carbone de la chaîne principale du polymère, notée----- représente une double liaison ou une simple liaison, conformément aux règles de valence de la chimie organique ;
- $R^1$, $R^2$, $R^3$, $R^4$, $R^5$, $R^6$, $R^7$ et $R^8$, identiques ou différents, représentent :

  - un atome d'hydrogène ou d'halogène ; ou
  - un radical comprenant de 1 à 22 atomes de carbone choisi parmi alkyle, alcényle, alcoxycarbonyle, alcényloxycarbonyle, alkylcarbonyloxy, alcénylcarbonyloxy, la chaîne hydrocarbonée dudit radical pouvant éventuellement être interrompue par au moins un atome d'oxygène ou un atome de soufre ; en outre:
  - au moins un des groupes $R^1$ à $R^8$ peut former, avec au moins un autre des groupes $R^1$ à $R^8$ et avec le ou les atomes de carbone auxquels lesdits groupes sont reliés, un cycle ou hétérocycle hydrocarboné saturé ou insaturé, éventuellement substitué, et comprenant de 3 à 10 chaînons; et
  - au moins une des paires ($R^1$, $R^2$), ($R^3$, $R^4$), ($R^5$, $R^6$) et ($R^7$, $R^8$) peut former, avec l'atome de carbone auquel ladite paire est reliée, un groupe carbonyle C=O ou un groupe de 2 atomes de carbone reliés par une double liaison : C=C, dont l'autre atome de carbone porte 2 substituants choisis parmi un atome d'hydrogène ou un radical $C_1$-$C_4$ alkyle ;

- x et y sont des nombres entiers, identiques ou différents, compris dans une fourchette allant de 0 à 6, la somme x + y étant comprise dans une fourchette allant de 0 à 6 ;
- $R^9$, $R^{10}$, $R^{11}$ et $R^{12}$, identiques ou différents, représentent :

  - un atome d'hydrogène ou d'halogène ; ou
  - un radical comprenant de 1 à 22 atomes de carbone et choisi parmi alkyle, alcényle, alcoxycarbonyle, alcényloxycarbonyle, alkylcarbonyloxy, alcénylcarbonyloxy, alkylcarbonyloxyalkyl, la chaîne hydrocarbonée dudit radical pouvant éventuellement être interrompue par au moins un atome d'oxygène ou un atome de soufre ; en outre:
  - au moins un des groupes $R^9$ à $R^{12}$ peut former, avec au moins un autre des groupes $R^9$ à $R^{12}$ et avec le ou les atomes de carbone auxquels lesdits groupes sont reliés, un cycle ou hétérocycle hydrocarboné saturé ou insaturé, éventuellement substitué, et comprenant de 3 à 10 chaînons; et
  - au moins une des paires ($R^9$, $R^{10}$) et ($R^{11}$, $R^{12}$) peut former avec l'atome de carbone auquel ladite paire est reliée un groupe de 2 atomes de carbone reliés par une double liaison : C=C, dont l'autre atome de carbone porte 2 substituants choisis parmi un atome d'hydrogène ou un radical $C_1$-$C_4$ alkyle ; et
  - l'atome de carbone porteur de l'un des groupes de la paire ($R^9$, $R^{10}$) peut être relié à l'atome de carbone porteur de l'un des groupes de la paire ($R^{11}$, $R^{12}$) par une double liaison, étant entendu que, conformément aux règles de valence, un seul des groupes de chacune de ces 2 paires est alors présent ;

- $R^{13}$ représente :

  - un atome d'oxygène ou de soufre, ou
  - un radical divalent $-CH_2-$, -C(=O)- ou $-NR^0-$ dans lequel $R^0$ est un radical alkyle ou alcényle comprenant de 1 à 22 atomes de carbone ;

- n1 et n2, identiques ou différents, sont chacun un nombre entier ou égal à 0, dont la somme est désignée par n ;
- m est un nombre entier ou égal à 0 ;
- p1 et p2, identiques ou différents, sont chacun un nombre entier ou égal à 0 dont la somme p1 + p2 vérifie l'équation : p1 + p2 = q x (z + 1) dans laquelle :

  - q est un nombre entier ou égal à 0 ; et
  - z est un nombre entier allant de 1 à 5 ; et

- n1, n2, m, p1 et p2 étant en outre tels que la masse moléculaire moyenne en nombre Mn du polymère de formule (I) est comprise dans une fourchette allant de 400 à 100 000 g/mol et son indice de polymolécularité est compris dans un domaine allant de 1,0 à 3,0.

**2.** Polymère hydrocarboné selon la revendication 1, **caractérisé en ce que** :

- les radicaux $R^{14}$ et $R^{15}$ représentent un radical $C_1$-$C_4$ alkyle ou bien forment avec l'atome de carbone auxquels ils sont reliés un radical cyclohexyle ;
- g et d représentent 0 ou 1 ;
- $R^1$, $R^2$, $R^3$, $R^4$, $R^5$, $R^6$, $R^7$ et $R^8$ représentent un atome d'hydrogène ou un radical alkyle comprenant de 1 à 14 atomes de carbone ;
- x et y sont compris dans une fourchette allant de 0 à 2, la somme x + y étant comprise dans une fourchette allant de de 0 à 2 ;
- $R^9$, $R^{10}$, $R^{11}$ et $R^{12}$ représentent un atome d'hydrogène ou un radical dont la partie hydrocarbonée comprend de 1 à 14 atomes de carbone ;
- $R^{13}$ représente le radical divalent $-CH_2-$,
- z est un nombre entier égal à 1 ou 2 ; et
- la masse moléculaire moyenne en nombre Mn est comprise dans une fourchette allant de 3000 à 50 000 g/mol.

**3.** Polymère hydrocarboné selon l'une des revendications 1 ou 2, **caractérisé en ce que** :

- lorsque m est non nul, que p1 et p2 sont non nuls et que n1 et n2 sont chacun égal à 0, alors le rapport :

$$m / (p1 + p2 + m)$$

est compris dans l'intervalle allant de 30 à 70 % ; ou
- lorsque m est égal à 0, que p1 et p2 sont non nuls et que la somme n1 + n2 est non nulle, alors au moins un des groupes $R^1$, $R^2$, $R^3$, $R^4$, $R^5$, $R^6$, $R^7$ et $R^8$ est autre qu'un atome d'hydrogène, et le rapport :

$$(n1 + n2) / (p1 + p2 + n1 + n2)$$

est compris dans l'intervalle allant de 30 à 70 % ; ou
- lorsque m est différent de 0, que p1 et p2 sont chacun égal à 0, que la somme n1 + n2 est non nulle, et que chacun des groupes $R^1$, $R^2$, $R^3$, $R^4$, $R^5$, $R^6$, $R^7$ et $R^8$ est un atome d'hydrogène, alors le rapport :

$$m / (m + n1 + n2)$$

est compris dans l'intervalle allant de 30 à 70 ; ou
- lorsque m est non nul, que p1 et p2 sont non nuls et que la somme n1 + n2 est non nulle et que chacun des groupes $R^1$, $R^2$, $R^3$, $R^4$, $R^5$, $R^6$, $R^7$ et $R^8$ est un atome d'hydrogène, alors le rapport :

$$m / ( p1 + p2 + n1 + n2 + m)$$

est compris dans l'intervalle allant de 30 à 70 % ; ou
- lorsque m est non nul, que p1 et p2 sont non nuls et que la somme n1 + n2 est non nulle et que au moins un des groupes $R^1$, $R^2$, $R^3$, $R^4$, $R^5$, $R^6$, $R^7$ et $R^8$ est autre qu'un atome d'hydrogène, alors le rapport :

$$(m + n1 + n2) / ( p1 + p2 + n1 + n2 + m)$$

est compris dans l'intervalle allant de 30 à 70 %.

**4.** Polymère hydrocarboné selon l'une des revendications 1 à 3, **caractérisé en ce qu'**il a la formule (I') suivante :

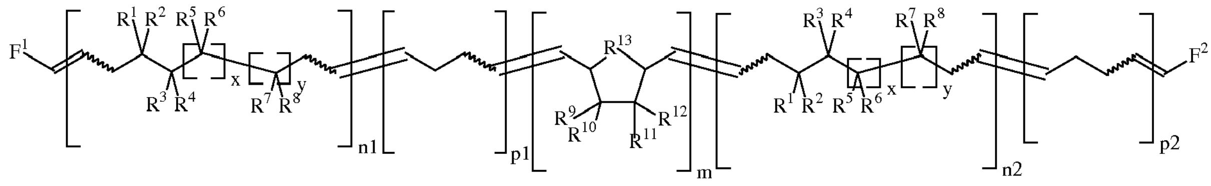

(I')

dans laquelle la liaison est une liaison orientée géométriquement d'un côté ou de l'autre par rapport à la double liaison.

**5.** Polymère hydrocarboné selon l'une des revendications 1 à 4, **caractérisé en ce qu'**il a la formule (II') suivante :

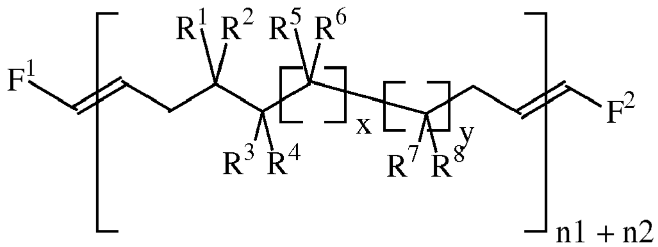

**6.** Polymère hydrocarboné selon l'une des revendications 1 à 4, **caractérisé en ce qu'**il a la formule (III') suivante :

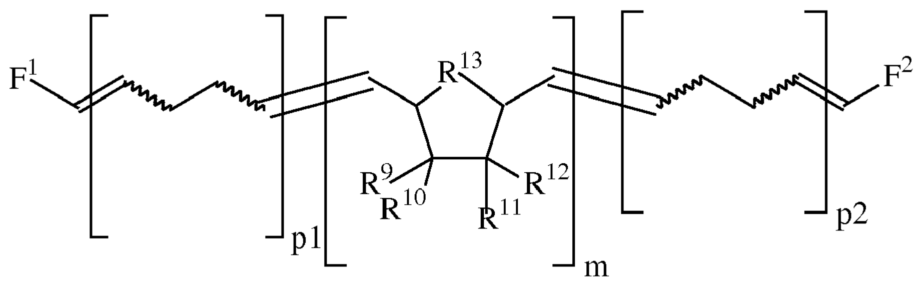

**7.** Procédé de préparation d'un polymère hydrocarboné comprenant deux groupements terminaux azlactones de formule (I), tel que défini dans l'une des revendications 1 à 6, ledit procédé comprenant au moins une réaction de polymérisation par ouverture de cycle par métathèse en présence :

(a) d'un catalyseur de métathèse ;
(b) d'un agent de transfert de chaîne (ou CTA) comprenant 2 groupements azlactones, de formule (B) suivante :

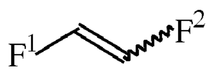
(B)

dans laquelle la liaison est une liaison orientée géométriquement d'un côté ou de l'autre par rapport à la double liaison ; et
(c) d'au moins un composé C choisi parmi :

- le composé de formule (C1) :

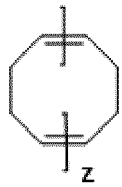

(C1) ;

- le composé de formule (C2) :

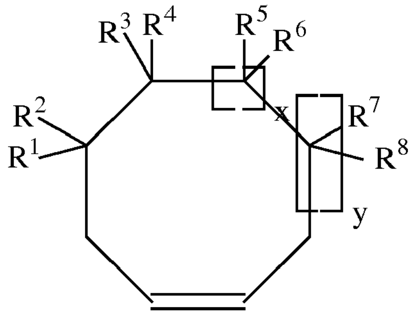

(C2) ;

et
- le composé de formule (C3) :

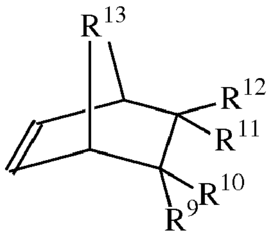

(C3)

ladite réaction de polymérisation étant mise en oeuvre :

- pendant une durée allant de 2 à 24 heures et à une température comprise dans un intervalle de 20 à 70 °C ; et
- avec un rapport r, égal au rapport du nombre de moles dudit CTA au nombre total de moles du composé C, compris dans un intervalle allant de 0,0010 à 1,0.

**8.** Utilisation comme adhésif du polymère hydrocarboné tel que défini dans l'une des revendications 1 à 6, en mélange en quantités stoechiométriques avec un composé aminé comprenant au moins deux groupements amines.

**9.** Procédé d'assemblage de deux substrats par collage, comprenant :

- l'enduction sur au moins un des deux substrats à assembler d'une composition adhésive liquide obtenue par mélange d'un composé aminé comprenant au moins deux groupements amines avec le polymère hydrocarboné tel que défini dans l'une des revendications 1 à 6 ; puis
- la mise en contact effective des deux substrats.

Europäisches Patentamt
European Patent Office
Office européen des brevets

# RAPPORT DE RECHERCHE EUROPEENNE

Numéro de la demande
EP 17 18 0763

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | US 2006/173145 A1 (PAWLOW JAMES H [US] ET AL) 3 août 2006 (2006-08-03)<br>* alinéas [0003], [0005], [0010] - [0018], [0025] - [0041], [0053], [0056], [0057], [0095] - [0102]; revendications 1,2,4-7; exemples 1-5 *<br>----- | 1-9 | INV.<br>C08G61/08<br>C08G61/12<br>C09J165/00<br>C09J177/00 |
| A | EP 0 128 731 A1 (MINNESOTA MINING & MFG [US]) 19 décembre 1984 (1984-12-19)<br>* page 8, ligne 23 - ligne 36 *<br>* page 10, ligne 29 - page 13, ligne 31 *<br>* revendications 1,6 *<br>----- | 1-9 | |
| A | EP 1 334 977 A1 (SEKISUI CHEMICAL CO LTD [JP]) 13 août 2003 (2003-08-13)<br>* alinéa [0062]; revendications 13,22 *<br>----- | 1-9 | |
| A | WO 2013/151739 A1 (3M INNOVATIVE PROPERTIES CO [US]; FORNOF ANN R [US]; CARUSO DAILEY MAR)<br>10 octobre 2013 (2013-10-10)<br>* exemples 5,16-19 *<br>----- | 1-9 | DOMAINES TECHNIQUES RECHERCHES (IPC) |
| A | VINCENT LAPINTE ET AL: "Synthesis and Ring-Opening Metathesis Polymerization(ROMP) Reactivity of endo- and exo-Norbornenylazlactone Using Ruthenium Catalysts",<br>MACROMOLECULAR CHEMISTRY AND PHYSICS.,<br>vol. 205, no. 6, 1 avril 2004 (2004-04-01), pages 824-833, XP055357141,<br>DE<br>ISSN: 1022-1352, DOI: 10.1002/macp.200300120<br>* schéma 2 *<br>----- | 1-9 | C08G<br>C09J<br>C08L<br>C09D |

Le présent rapport a été établi pour toutes les revendications

1

| Lieu de la recherche | Date d'achèvement de la recherche | Examinateur |
|---|---|---|
| Munich | 16 novembre 2017 | Meiners, Christian |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

## ANNEXE AU RAPPORT DE RECHERCHE EUROPEENNE RELATIF A LA DEMANDE DE BREVET EUROPEEN NO.

EP 17 18 0763

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

16-11-2017

| Document brevet cité au rapport de recherche | Date de publication | Membre(s) de la famille de brevet(s) | Date de publication |
|---|---|---|---|
| US 2006173145 A1 | 03-08-2006 | AUCUN | |
| EP 0128731 A1 | 19-12-1984 | AU 565743 B2 | 24-09-1987 |
| | | BR 8402880 A | 21-05-1985 |
| | | CA 1243558 A | 25-10-1988 |
| | | DE 3466239 D1 | 22-10-1987 |
| | | EP 0128731 A1 | 19-12-1984 |
| | | JP H0613680 B2 | 23-02-1994 |
| | | JP S6011515 A | 21-01-1985 |
| | | ZA 8404053 B | 29-01-1986 |
| EP 1334977 A1 | 13-08-2003 | AU 8252301 A | 13-03-2002 |
| | | CA 2420839 A1 | 27-02-2003 |
| | | CN 1449403 A | 15-10-2003 |
| | | EP 1334977 A1 | 13-08-2003 |
| | | JP 2003081990 A | 19-03-2003 |
| | | KR 20030059118 A | 07-07-2003 |
| | | TW 553947 B | 21-09-2003 |
| | | US 2004015002 A1 | 22-01-2004 |
| | | WO 0218399 A1 | 07-03-2002 |
| WO 2013151739 A1 | 10-10-2013 | CN 104254581 A | 31-12-2014 |
| | | EP 2834313 A1 | 11-02-2015 |
| | | JP 2015518504 A | 02-07-2015 |
| | | KR 20140142340 A | 11-12-2014 |
| | | US 2015232596 A1 | 20-08-2015 |
| | | WO 2013151739 A1 | 10-10-2013 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2014091173 A
- EP 0105665 A
- US 5849851 A
- WO 200104173 A
- WO 2011038057 A


### Littérature non-brevet citée dans la description


- **MARUOKA, K.** *Angew. Chem. Int. Ed.,* 2007, vol. 46, 4222
- **OOI, T. ; MARUOKA, K.** *Aldrichmica Acta,* 2007, vol. 40, 77
- **ABOUL-ENEIN, M.N. ; EL-AZZOUNY, A. A. ; ABDALLAH, N. A. ; MAKHLOUF, A. A.** *Egyptian Journal of Chemistry,* 1991, vol. 34 (6), 549-558